(19) Europäisches Patentamt
European Patent Office
Office européen des brevets

(11) EP 2 769 708 A1

(12) **EUROPÄISCHE PATENTANMELDUNG**

(43) Veröffentlichungstag:
**27.08.2014 Patentblatt 2014/35**

(51) Int Cl.:
***A61K 8/44*** *(2006.01)* ***A61Q 17/04*** *(2006.01)*

(21) Anmeldenummer: **13156181.3**

(22) Anmeldetag: **21.02.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Symrise AG**
**37603 Holzminden (DE)**

(72) Erfinder:
- **Vielhaber, Gabriele**
**75008 Paris (FR)**
- **Le Maire, Marielle**
**92100 Boulogne (FR)**

(54) **Inhibition der durch IR-Strahlung induzierten Hautalterung**

(57) Die Erfindung betrifft ein Medikament enthaltend mindestens eine Verbindung der Formel (I)

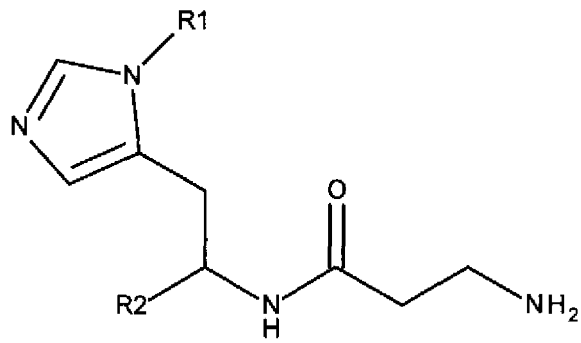

mit der Maßgabe, dass R1 für H oder $CH_3$ und R2 für H oder COOH steht, oder deren Salze zur Inhibierung der durch Infrarotsrahlung induzieren Hautalterung.

EP 2 769 708 A1

## Beschreibung

### Gebiet der Erfindung

Die Erfindung befindet sich auf dem Gebiet der pharmazeutischen und kosmetischen Wirkstoffe und betrifft neue Medikamente speziell zur Inhibierung durch IR-Strahlung induzierten Hautalterung, Medikamente sowie Zubereitungen, die diese Stoffe enthalten, nichttherapeutische Verfahren zur topischen Behandlung von Haut und Haaren sowie die Verwendung der Stoffe als Inhibitoren für die durch IR-Strahlung induzierte Hautalterung.

### Stand der Technik

Es wird seit langer Zeit angestrebt, die Hautalterung anzuhalten oder zumindest zu verzögern. Die Wissenschaft konnte als Ursachen der Hautalterung verschiedenste Beschleuniger aufzeigen, beispielsweise Schadstoffe aus der Umwelt, freie Radikale, oxidativen Stress, Entzündungen, ultraviolette Strahlung (UV-Strahlung) oder Infrarotstrahlung (IR-Strahlung).

Es werden ferner verschiedene Mechanismen der Hautalterung diskutiert, wobei sehr häufig enzymatische Mechanismen genannt werden und dem Enzym "Matrix-Metallo-Proteinase 1" (MMP-1) eine besondere Bedeutung zugesprochen wird. Eine vermehrte Expression oder Aktivierung von MMP-1 bzw. eine Steigerung der MMP-1-Aktivität werden im Allgemeinen als Beschleuniger der Hautalterung angesehen.

UV-Strahlung und IR-Strahlung werden als Beschleuniger der Hautalterung angesehen, wobei deren Wirkung über verschiedenste Mechanismen hervorgerufen wird, etwa durch Schädigung der Erbsubstanz, Proteindenaturierung oder durch Induktion der MMP-1-Expression. Es gibt bisher sehr gute Möglichkeiten, die durch UV-Strahlung hervorgerufene Hautalterung z.B. durch die Verwendung von UV-Lichtschutzfaktoren zu vermindern, jedoch mangelt es an vielfältigen Möglichkeiten oder Alternativen die durch IR-Strahlung hervorgerufene Hautalterung zu stoppen oder zumindest zu reduzieren.

IR-Strahlung ist nicht nur ein Hauptbestandteil des Sonnenlichts, sondern entsteht auch bei vielfältigen technischen Prozessen und ist insofern von großer Bedeutung bei der Alterung der Haut, wobei insbesondere der IRA-Bereich als besonders kritisch angesehen wird. IRA-Strahlung kann sehr tief in die Haut eindringen, wobei sogar ein Großteil der IRA-Strahlung durch die Epidermis hindurch gelangt und in die darunter befindliche Dermis eindringt.

In diesem Zusammenhang sei auf die WO 2010124817 A2 (Kao) hingewiesen. Die Schrift betrifft Zubereitungen mit einem Gehalt an Dipeptiden. In den Bespielen 14 bis 16 werden Haarbehandlungsmittel offenbart, die auch L-Carnosin, jedoch keine Lichtschutzfaktoren enthalten.

Gegenstand der DE 10300782 A1 (Beiersdorf) sind multiple Emulsionen mit einem Gehalt an Silikonverbindungen, die optional auch Carnotin oder Carnosin enthalten können. Die Zubereitungen weisen keine weiteren Lichtschutzfaktoren auf.

WO 2007122822 A1 (Shiseido) betrifft kosmetische Sonnenschutzemulsionen. In den Beispielen 22 und 23 werden Formulierungen offenbart, die auch Carnosin zusammen mit weiteren Lichtschutzfaktoren wie z.B. 5 Gew.-% Octocrylen enthalten. Die Schrift enthält jedoch keinen Hinweis auf die Eigenschaft von Carnosin, IR induzierte Hautalterung zu inhibieren.

Es war daher Aufgabe der vorliegenden Erfindung, die durch IR-Strahlung verursachte Hautalterung zu reduzieren oder zu verhindern, entsprechende Wirkstoffe dafür anzugeben und spezielle Formulierungen zur zielgerichteten Applikation der Wirkstoffe bereitzustellen. Neben einer sehr hohen Effektivität der Wirkstoffe weisen diese eine sehr gute Verträglichkeit auf, führen nicht zur Hautrötung, Hautbleichung oder Hautbräunung, sind nicht irritierend, trocknen die Haut nicht aus, bilden keine feuchten, schuppigen, pudrigen oder klebrigen Filme darauf und machen die Haut nicht rissig. Die erfindungsgemäßen Zusammensetzungen sollten neben einem zielgerichteten Transport der erfindungsgemäßen Wirkstoffe zum Wirkungsort unter anderem dafür sorgen, dass die erfindungsgemäßen Wirkstoffe den Wirkungsort nach dem Auftragen sehr schnell erreichen und diese langanhaltend an den Wirkungsort abgegeben werden.

### Beschreibung der Erfindung

Ein erster Gegenstand der Erfindung betrifft ein Medikament enthaltend mindestens eine Verbindung der Formel (I)

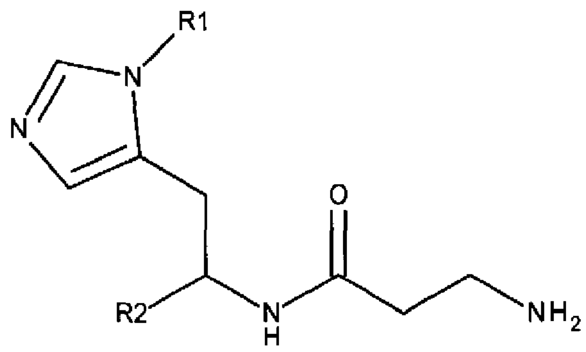

mit der Maßgabe, dass R1 für H oder $CH_3$ und R2 für H oder COOH steht, oder deren Salze, vorzugsweise zur Inhibierung der durch Infrarotstrahlung induzierten Hautalterung und besonders bevorzugt zur Inhibierung der durch Infrarotstrahlung induzierten MMP-1-Expression, zur Inhibierung der durch IR-Strahlung induzierten Synthese der MMP-1 codierenden mRNA, zur Inhibierung der durch IR-Strahlung induzierten Translation der MMP-1 aus der mRNA oder / und zur Inhibierung der durch IR-Strahlung induzierten MMP-1 Aktivierung.

Überraschenderweise wurde gefunden, dass die Verbindungen der Formel (I)sowie deren Salze in signifikanter Weise die durch IR-Strahlung induzierte MMP-1 in menschlichem oder tierischem Gewebe oder/und in oder an den Fibroblasten der Dermis (dermale Fibroblasten) inhibieren.

Die Expression von MMP-1 läuft nahezu stetig in Hautzellen ab. Das dabei gebildete Enzym MMP-1 baut Bindegewebe ab und ist somit ein Teil des notwendigen Erneuerungsprozesses der Haut. Durch IR-Bestrahlung nimmt die Expression von MMP-1 deutlich zu und führt zu einem unerwünscht hohen Abbau von Bindegewebe und in dessen Folge zur Beschleunigung der Hautalterung. Unter der Inhibierung der durch Infrarotstrahlung induzierten MMP-1-Expression wird erfindungsgemäß die Verminderung der durch die Infrarotstrahlung verursachte vermehrte MMP-1-Expression verstanden. Unter der Inhibierung der durch Infrarotstrahlung induzierten Hautalterung wird erfindungsgemäß die Verminderung der durch die Infrarotstrahlung verursachte vermehrte Hautalterung verstanden.

In Kombination mit einer Vielzahl unterschiedlicher UV-Lichtschutzfaktoren verbessern die Verbindungen der Formel (I) zudem den Schutz gegen schädliche Einflüsse der Sonnenstrahlung auf menschliche Haut und Haare in synergistischer Weise.

**Wirkstoffe**

Bei den Wirkstoffen, die die Formel (I) ausmachen, handelt es sich um grundsätzlich bekannte Verbindungen, die nach den üblichen Verfahren der organischen Chemie zugänglich sind. Vorzugsweise handelt es sich dabei um Gruppe von Stoffen, die gebildet wird von Carnosin, L-Carnosin, D-Carnosin, D/L-Carnosin, Carnicin, Carnicin*2HCl, Anserin, D-Anserin, L-Anserin sowie L-Anserin*$HNO_3$ und deren Mischungen.

Unter Salzen der Verbindungen der Formel (I) werden erfindungsgemäß bevorzugt Salze der Verbindungen der Formel (I) mit mineralischen Säuren verstanden und besonders bevorzugt Salze der Formel (II):

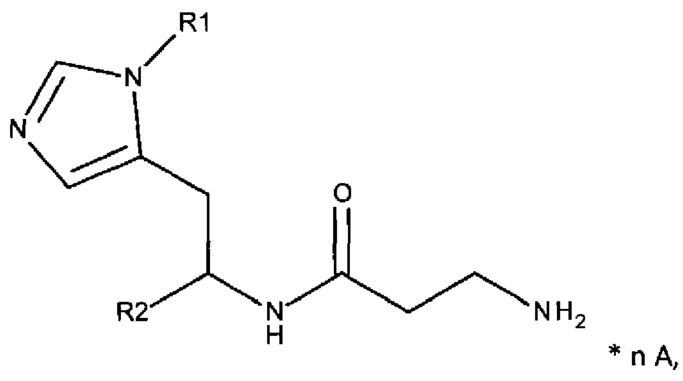

wobei n für 1,2 oder 3 und A für HCl oder $HNO_3$ steht und R1 für H oder CH3 sowie R2 für H oder COOH.

Unter IR-Strahlung (Abkürzung für Infrarotstrahlung) wird erfindungsgemäß eine elektromagnetische Strahlung im Wellenlängenbereich von 0,76 μm bis 1000 μm verstanden. Bevorzugt werden darunter die NIR-Strahlung mit einem Wellenlängenbereich von 0,76 bis 3,0 μm und ganz besonders bevorzugt die IR-A-Strahlung im Wellenlängenbereich

von 0,76 bis 1,4 µm verstanden. Insbesondere bevorzugt wird unter dem Begriff IR-Strahlung eine IR-A-Strahlung mit einer Strahlungsdosis von 10-1000 J/$cm^2$ und ganz besonders bevorzugt eine IR-A-Strahlung mit einer Strahlungsdosis von 100-400 J/$cm^2$ verstanden. Insbesondere in diesen Dosisbereichen wirken die Verbindungen der Formel (I) oder deren Salze besonders gut.

Die erfindungsgemäße Wirkung kann beispielsweise durch Bestrahlung von Haut oder humanen dermalen Fibroblasten mit IR-A Strahlung einer Intensität von 360 mW/$cm^2$ und einer Dosis von 360 J/$cm^2$ simuliert werden.

In weiteren Ausgestaltungsformen der Erfindung finden die Verbindungen der Formel (I) oder deren Salze Verwendung zur Inhibierung der durch IR-Strahlung induzierten Hautalterung, bevorzugt zur Inhibierung der durch IR-Strahlung induzierten MMP-1 Expression, zur Inhibierung der durch IR-Strahlung induzierten Synthese der MMP-1 codierenden mRNA, zur Inhibierung der durch IR-Strahlung induzierten Translation der MMP-1 aus der mRNA oder/und zur Inhibierung der durch IR-Strahlung induzierten MMP-1 Aktivierung, wobei die Inhibierung jeweils bevorzugt in oder an den Fibroblasten der Dermis (dermale Fibroblasten) erfolgt.

**Pharmazeutische Zubereitungen**

Ein weiterer Gegenstand der Erfindung betrifft pharmazeutische Zubereitungen, enthaltend

(a) mindestens eine Verbindung der Formel (I) oder deren Salz,

(b) mindestens einen UV-Lichtschutzfaktor sowie

(c) einen pharmazeutisch zulässigen Träger.

Die Zubereitungen können als Verbindungen der Formel (I) die oben angegebenen Stoffe enthalten, wobei das Gewichtsverhältnis der Komponenten (a) und (b) zueinander etwa 1:99 bis etwa 99:1, vorzugsweise etwa 10:90 bis etwa 90:10, weiter bevorzugt etwa 20:80 bis etwa 80:20 und insbesondere bevorzugt etwa 40:60 bis etwa 60:40 betragen kann. Der Gewichtsanteil der Komponenten (a+b) bezogen auf den Träger kann etwa 0,5 bis etwa 50, vorzugsweise etwa 1 bis etwa 30, weiter bevorzugt etwa 2 bis etwa 10 und insbesondere bevorzugt etwa 2,5 bis etwa 5 Gew.-% betragen.

Die Zubereitungen können dabei insbesondere als Cremes, Lotionen, Gele, Pasten oder Kapseln vorliegen.

**Kosmetische Zubereitungen**

Eine weitere Ausgestaltungsform der Erfindung betrifft kosmetische Zubereitungen, enthaltend

(a) mindestens eine Verbindung der Formel (I) oder deren Salz,

(b) mindestens einen UV-Lichtschutzfaktor sowie

(c) einen kosmetisch zulässigen Träger.

Auch diese Zubereitungen können als Verbindungen der Formel (I) die oben angegebenen Stoffe enthalten, wobei das Gewichtsverhältnis der Komponenten (a) und (b) zueinander wiederum etwa 1:99 bis etwa 99:1, vorzugsweise etwa 10:90 bis etwa 90:10, weiter bevorzugt etwa 20:80 bis etwa 80:20 und insbesondere bevorzugt etwa 40:60 bis etwa 60:40 betragen kann. Der Gewichtsanteil der Komponenten (a+b) bezogen auf den Träger kann ebenfalls etwa 0,5 bis etwa 50, vorzugsweise etwa 1 bis etwa 30, weiter bevorzugt etwa 2 bis etwa 10 und insbesondere bevorzugt etwa 2,5 bis etwa 5 Gew.-% betragen.

**UV-Lichtschutzfaktoren**

Mischungen von Verbindungen der Formel (I) oder deren Salzen mit UV-Lichtschutzfaktoren führt zu einer synergistischen Verstärkung des Schutzes von Haut und Haaren gegen die schädlichen Einwirkung von Sonnenlicht. Bei den UV-Lichtschutzfaktoren kann es sich dabei um UV-A-Filter, UV-B-Filter, Pigmente oder deren Gemische handeln, die im Folgenden weiter erläutert sind:

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzylidenjcampher beschrieben;

- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;

- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);

- Ester der Salicylsäure, vorzugsweise Salicylsäure-2-ethylhexylester, Salicylsäure-4-isopropylbenzylester, Salicylsäurehomomenthylester;

- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

- Ester der Benzalmalonsäure, vorzugsweise 4-Methoxybenzmalonsäuredi-2-ethylhexylester;

- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);

- Propan-1,3-dione, wie z.B. 1-(4-tert.Butylphenyl)-3-(4'methoxyphenyl)propan-1,3-dion;

- Ketotricyclo(5.2.1.0)decan-Derivate.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;

- 1H-Benzimidazole-4,6-Disulfonic Acid, 2,2'-(1,4-Phenylene)Bis-, Disodium Salt (Neo Heliopan® AP)

- Sulfonsäurederivate von Benzophenonen, vorzugsweise 2-Hydroxy-4-methoxybenzophenon-5-sulfonsäure und ihre Salze;

- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxy-dibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäure-isoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Eine bevorzugte Ausführungsform besteht darin, dass im Fall, dass die Verbindung (I) Carnosin und der Lichtschutzfaktor Octocrylen ist, letzteres nur in Mengen von unter 5 Gew.-% - bezogen auf die Zusammnsetzung - vorhanden sein darf.

Neben den genannten löslichen Stoffen kommen für diesen Zweck auch unlösliche Lichtschutz-pigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt

abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul $TiO_2$ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

**Pharmazeutisch und kosmetisch zulässige Träger**

Sowohl die pharmazeutischen als auch die kosmetischen Zubereitungen können als Komponente (c) Träger bzw. Lösungsmittel enthalten, die ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylenglycol(2)methylether, Propylpropyl-englycol(3)methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkane, C14-C17 Alkane, C11-C15 Cycloalkane, Caprylylbutyrat, Isoparaffine, Diacetin, Triacetin Dicaprylylether, Dicaprylylmaleat, und deren Mischungen.

**Zusatzstoffe**

In einer bevorzugten Ausführungsform können sowohl die pharmazeutischen als auch die kosmetischen Zubereitungen weitere Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Silikonverbindungen, Fette, Wachse, Lecithine, Phospholipide, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen. Insbesondere enthalten diese Zubereitungen weiterhin Ölkörper und/oder Emulgatoren.

**Kosmetische und/oder pharmazeutische Mittel**

Die erfindungsgemäßen kosmetischen und/oder pharmazeutischen Mittel können weitere typische Hilfs- und Zusatzstoffe enthalten, wie beispielsweise milde Tenside, Ölkörper, Emulgatoren, Perlglanzwachse, Kühlstoffe, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

**1. Tenside**

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenvertellung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

**2. Ölkörper**

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

**3. Emulgatoren**

Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;

- Alkyl- und/oder Alkenyloligoglykoside mit 8 bis 22 Kohlenstoffatomen im Alk(en)ylrest und deren ethoxylierte Analoga;

- Anlagerungsprodukte von 1 bis 15 Mol Ethylenoxid an Ricinusöl und/oder gehärtetes Ricinusöl;

- Anlagerungsprodukte von 15 bis 60 Mol Ethylenoxid an Ricinusöl und/oder gehärtetes Ricinusöl;

- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;

- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren

mit 3 bis 18 Kohlenstoffatomen sowie deren Addukte mit 1 bis 30 Mol Ethylenoxid;

- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

- Mono-, Di- und Trialkylphosphate sowie Mono-, Di- und/oder Tri-PEG-alkylphosphate und deren Salze;

- Wollwachsalkohole;

- Polysiloxan-Polyalkyl-Polyether-Copolymere bzw. entsprechende Derivate;

- Block-Copolymere z.B. Polyethylenglycol-30 Dipolyhydroxystearate;

- Polymeremulgatoren, z.B. Pemulen-Typen (TR-1,TR-2) von Goodrich oder Cosmedia® SP von Cognis;

- Polyalkylenglycole sowie

- Glycerincarbonat.

Im Folgenden werden besonders geeignete Emulgatoren näher erläutert:

(i) **Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind als Rückfettungsmittel für kosmetische Zubereitungen bekannt.

(ii) **Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

(iii) **Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

(iv) **Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

(v) **Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform@ TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate

T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

(vi) **Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

(vii) **Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Deraat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das $C_{12/18}$-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

## 4. Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

## 5. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

## 6. Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS[1] 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

[1] FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

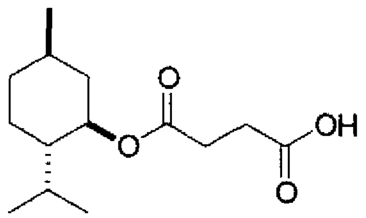
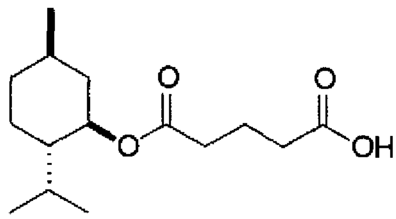

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

## 7. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### 8. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### 9. Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylatetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/vinylcaprolactam-Terpolymere sowie gegebenenfalls derivatisierte Celluloseether und Silicone in Frage.

### 10. Silikonverbindungen

Geeignete Silikonverbindungen sind beispielsweise Dimethylpolysiloxane, Methylphenylpolysiloxane, cyclische Silikone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### 11. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

## 12. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate

(Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

## 13. Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

## 14. Antischuppenwirkstoffe

Als Antischuppenwirkstoffe kommen Piroctón Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyllpiperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

## 15. Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

## 16. Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

**17. Hydrotrope**

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;

- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%;

- Methyolverbindungen, wie insbesondere Trimethylolethan, Trimethylolpropan, Trimethylolbutan, Pentaerythrit und Dipentaerythrit;

- Niedrigalicylglucoside, insbesondere solche mit 1 bis 8 Kohlenstoffen im Alkylrest, wie beispielsweise Methyl- und Butylglucosid;

- Zuckeralkohole mit 5 bis 12 Kohlenstoffatomen, wie beispielsweise Sorbit oder Mannit,

- Zucker mit 5 bis 12 Kohlenstoffatomen, wie beispielsweise Glucose oder Saccharose;

- Aminozucker, wie beispielsweise Glucamin;

- Dialkoholamine, wie Diethanolamin oder 2-Amino-1,3-propandiol.

**18. Konservierungsmittel**

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

**19. Parfümöle und Aromen**

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\alpha$-Isomethylionon und Methylcedrylketon, zu den Alkoholen A-nethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl,

Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

**20. Farbstoffe**

Als Farbstoffe können die für kosmetische Zwecke geeigneten und zugelassenen Substanzen verwendet werden, wie sie beispielsweise in der Publikation **"Kosmetische Färbemittel"** der **Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Auch die kosmetischen Zubereitungen können als Cremes, Lotionen, Ölformulierungen, Emulsionen, Gele, Pasten, Sprays, oder Kapseln vorliegen, wobei es sich dabei Hautpflegemittel, Sonnenschutzmittel oder Haarpflegemittel handelt.

Die erfindungsgemäßen Zusammensetzungen ermöglichen einen schnellen und zielgerichteten Transport der Verbindungen der Formel (I) nach Anspruch 1 oder deren Salze von der Hautoberfläche zu den dermalen Fibroblasten oder in die dermalen Fibroblasten. Sie ermöglichen insbesondere eine Inhibierung der durch Infrarotstrahlung induzierten MMP-1-Expression direkt oder kurzzeitig nach dem Auftragen der erfindungsgemäßen Zusammensetzung. Die Wirkung setzt bevorzugt innerhalb von 24 Stunden und besonders bevorzugt innerhalb von 12 oder 6 Stunden nach dem Auftragen der Zusammensetzungen ein.

Die erfindungsgemäßen Zusammensetzungen ermöglichen nach dem Auftragen auf die Haut eine besonders langanhaltende Inhibierung der durch Infrarotstrahlung induzierten MMP-1-Expression. Die erfindungsgemäße Wirkung lässt sich bei einmaligem Auftragen bevorzugt mindestens 6 Stunden, bevorzugt mindestens 12 Stunden und besonders bevorzugt mindestens 24 Stunden nachweisen.

In einer besonderen Ausführungsform der Erfindung ermöglichen die erfindungsgemäßen Zusammensetzungen sowohl eine schnelle und auch eine langanhaltende erfindungsgemäße Wirkung.

Zu den besonderen Vorteilen der erfindungsgemäßen Zusammensetzungen zählt, dass diese den schnellen, zielgerichteten und/oder langanhaltenden Transport der Verbindungen der Formel (I) an oder in die Fibroblasten auch während oder nach einer längeren Bestrahlung (bevorzugt mindestens 1 Stunde, weiter bevorzugt mindestens 6 oder 12 Stunden) mit IR-Strahlung gewährleisten können.

Die Erfindungsgemäßen Zusammensetzungen weisen bevorzugt einen pH-Wert von 5-8 und besonders bevorzugt von 5-7 auf.

Ein weiterer Gegenstand der vorliegenden Erfindung betrifft ein nicht-therapeutisches Verfahren zur Inhibierung der durch Infrarotstrahlung induzierten Hautalterung, bevorzugt zur Inhibierung der durch Infrarotstrahlung induzierten MMP-1-Expression, bei dem eine wirksame Menge einer Verbindung der Formel (I) oder deren Salze mit der menschlichen Haut oder den Haaren in Kontakt gebracht wird. Eine wirksame Menge ist beispielsweise dann erreicht, wenn eine kosmetische Zubereitung zur topischen Anwendung gebracht wird, die die Verbindungen der Formel (I) -gegebenenfalls zusammen mit UV-Lichtschutzfaktoren - in Mengen von 0,1 bis 5 Gew.-% enthalten.

Schließlich betrifft die Erfindung auch die Verwendung von Verbindungen der Formel (I) oder deren Salze zur Inhibierung der durch IR-Strahlung induzierten Hautalterung, bevorzugt zur Inhibierung der durch IR-Strahlung induzierten MMP-1 Expression, zur Inhibierung der durch IR-Strahlung induzierten Synthese der MMP-1 codierenden mRNA, zur Inhibierung der durch IR-Strahlung induzierten Translation der MMP-1 aus der mRNA oder / und zur Inhibierung der durch IR-Strahlung induzierten MMP-1 Aktivierung.

**Beispiele**

**Beispiele 1 und 2, Vergleichsbeispiele V1 und V2**

Humane dermale Fibroblasten (HDF) der neonatalen Vorhaut wurden in DMEM (Dulbecco's Modified Eagle Medium, PAA Laboratories GmbH, Kolbe, Deutschland) in 6-Well-Platten mit 5% fötalem Kälberserum (FKS, Invitrogen, Karlsruhe, Deutschland), 1% L-Glutamin, 1% nichtessentiellen Aminosäuren (Invitrogen, Karlsruhe, Deutschland), 1%

Streptomycin / Amphotericin B (Invitrogen, Karlsruhe, Deutschland) in einer befeuchteten Atmosphäre mit 5% CO2 für 4 Tage bis zur Konfluenz kultiviert. (wie beschrieben in Schroeder P, Lademann J. Darvin ME, Stege H, Marks C, Bruhnke S, Krutmann J (2008) Infrared radiation-induced matrix metalloproteinase in human skin. Implications for protection. J Invest Dermatol 128: 2491-2497)

Für die folgenden Untersuchungen wurden Fibroblasten der frühen Passage (<12) selektiert und verwendet. Nach Erreichen der Konfluez wurden die Zellen für 24 Stunden mit Medium ohne FKS kultiviert, wobei 0,001% und 0,0001% L-Carnosin aufgetragen wurde. Anschließend wurde das Medium durch PBS ausgetauscht und die offenen 6-Well-Platten mit IR-A-Strahlung einer Intensität von 360 $mW/cm^2$ und einer Dosis von 360 $J/cm^2$ bestrahlt. Hierzu wurde ein Hydrosun 500H IRA Gerät der Hydrosun Medizintechnik GmbH in Müllheim, Deutschland mit einem gekühlten Probentisch verwendet. Nach der Bestrahlung wurde das PBS durch ein Kulturmedium mit 2% FKS ausgetauscht und L-Carnosin wiederum in verschiedenen Konzentrationen (0,001 Gew.-% und 0,0001Gew.-%) aufgetragen. Zu Kontrollzwecken wurde parallel zu den Inkubationen mit L-Carnosin jeweils eine Kontrolle mit 1% DM50 mitgeführt. Die Zellen wurden für weitere 24 Stunden bis zur Gewinnung der RNA kultiviert. Die MMP-1 Genexpression wurde mittels quantitativer Real Time PCR (qRT-PCR) erfasst. 18S diente als Housekeeping Gen. Die Auswertung erfolgte mittels $\Delta\Delta C_T$-Methode.

<u>Verwendete Primer-Paare:</u>

| Marker | Sequenz |
|---|---|
| 18S | 5'-GCCGCTAGAGGTGAAATTCTTG-3' |
| | 5'-CATTCTTGGCAAATGCTTTCG'-3' |
| MMP-1 | 5'-CATGAAAGGTGGACCAACAATTT-3' |
| | 5'-CCAAGAGAATGGCCGAGTTC-3' |

Die Inhibition der IR-induzierten MMP-1-Expression (IH) wurde berechnet nach der Formel:

IH = [RQ (c mit Bestrahlung) – RQ (c0 ohne Bestrahlung)] / [RQ (c0 mit Bestrahlung – RQ (c0 ohne Bestrahlung)]

Wobei gilt:

| | |
|---|---|
| IH | Inhibition der IR-induzierten MMP-1-Expression |
| RQ (c0 mit Bestrahlung) | MMP-1 Expression nach IR-Bestrahlung ohne Wirkstoff |
| RQ ($c_0$ ohne Bestrahlung) | MMP-1 Expression ohne IR-Bestrahlung ohne Wirkstoff |
| RQ (c mit Bestrahlung) | MMP-1 Expression nach IR-Bestrahlung mit Zusatz des Wirkstoffs in der Konzentration c |

Die Ergebnisse sind in **Tabelle 1** zusammengefasst:

**Tabelle** 1

| Inhibierung der MMP-1 Expression | | | |
|---|---|---|---|
| **Zubereitung** | | **RQ MMP-1** | **Inhibition der IR-induzierten MMP-1 Expression (IH)** |
| **Kontrolle: ohne Zusatz von L-Carnosin** | | | |
| V1 | 0 Gew.-% ohne IR-A Bestrahlung | 1 | - |
| V2 | 0 Gew.-% mit IR-A Bestrahlung | 4,6 | - |
| **Mit Zusatz von L-Carnosin** | | | |
| 1 | 0,0001 Gew.-% mit IR-A Bestrahlung | 3,60 | 28% |
| 2 | 0,001 Gew.-% mit IR-A Bestrahlung | 2,1 | 69% |

Die Messergebnisse zeigen, dass der Zusatz von L-Carnosin wirksam die durch Infrarotstrahlung induzierte MMP-1 Expression inhibiert.

**Beispiele 3 bis 6, Vergleichsbeispiel V3**

Inhibierung der MMP-1 Synthese

Untersucht wurde die Fähigkeit der Testsubstanzen den toxischen Einfluss von UVA-Strahlen zu mindern. Als in-vitro System diente eine Kultur dermaler Fibroblasten, bestimmt wurde die Freisetzung von MMP-1 aus diesen Fibroblasten unter dem Einfluss der UV-Strahlung. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem $CO_2$-Level von 5 Vol.-% wurde das Nährmedium durch eine Elektrolytlösung ersetzt und die Fibroblasten mit einer definierten UVB-Strahlungsmenge geschädigt (50 $mJ/cm^2$). Die Bestimmung der MMP erfolgte mit einem Kit, das unter der Bezeichnung RPN2610 von der Firma Amersham im Handel erhältlich ist. Die Ergebnisse sind in **Tabelle 2** zusammengefasst. Angegeben ist die Menge MMP in ng/ml aus einer Testreihe mit Dreifach bestimmung.

**Tabelle 2**

| MMP-Inhibierung (Angabe in %-rel.) | | | | |
|---|---|---|---|---|
| **Bsp.** | **Testprodukt** | **Konz. % w/v** | **MMP** | |
| | | | **ohne UVA** | **mit UVA** |
| V3 | Kontrolle | - | 49±9 | 199±25 |
| 3 | D/L-Carnosin | 0,001 | 22±5 | 101±17 |
| 4 | Carnicin*2HCl | 0,001 | 24±7 | 118±21 |
| 5 | L-Carnosin + Uvinul A (1:1) | 0,001 | 12±4 | 88±12 |
| 6 | L-Carnosin + Octocrinyl (1:1) | 0,001 | 14±3 | 92±15 |

Die Ergebnisse zeigen, dass die Testsubstanzen die Freisetzung von MMP bei UVA-Bestrahlung nachhaltig vermindern.

## Patentansprüche

1. Medikament enthaltend mindestens eine Verbindung der Formel (I)

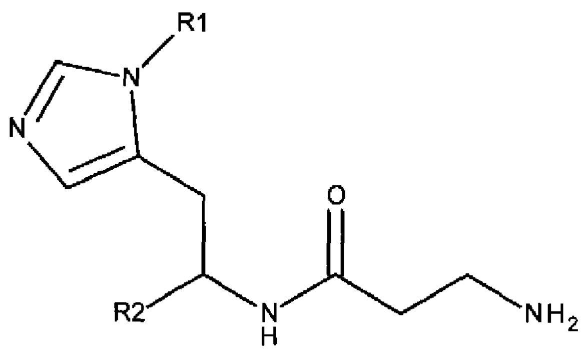

mit der Maßgabe, dass R1 für H oder $CH_3$ und R2 für H oder COOH steht, oder deren Salze.

2. Medikament enthaltend mindestens eine Verbindung der Formel (I)

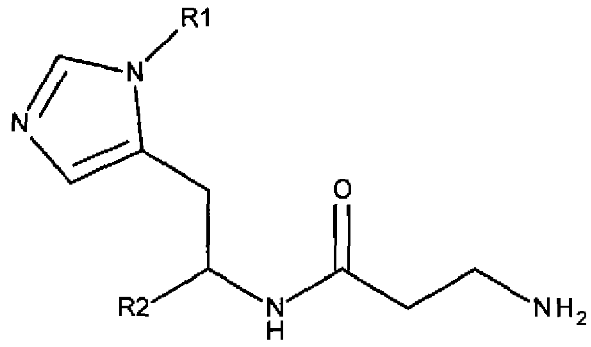

mit der Maßgabe, dass R1 für H oder $CH_3$ und R2 für H oder COOH steht, oder deren Salze zur Inhibierung der durch Infrarotstrahlung induzierten Hautalterung.

3. Medikament nach den Ansprüchen 1 oder 2, **dadurch gekennzeichnet, dass** die Verbindung der Formel (I) ausgewählt ist aus der Gruppe die gebildet wird von Carnosin, L-Carnosin, D-Carnosin, D/L-Carnosin, Carnicin, Carnicin*2HCl, Anserin, D-Anserin, L-Anserin sowie L-Anserin*$HNO_3$ und deren Mischungen.

4. Pharmazeutische Zubereitungen, enthaltend

   (a) mindestens eine Verbindung der Formel (I) oder deren Salz,
   (b) mindestens einen UV-Lichtschutzfaktor sowie
   (c) einen pharmazeutisch zulässigen Träger.

5. Zubereitungen nach Anspruch 4, **dadurch gekennzeichnet, dass** sie als Cremes, Lotionen, Gele, Pasten oder Kapseln vorliegen.

6. Kosmetische Zubereitungen, enthaltend

   (a) mindestens eine Verbindung der Formel (I) oder deren Salz,
   (b) mindestens einen UV-Lichtschutzfaktor sowie
   (c) einen kosmetisch zulässigen Träger.

7. Zubereitungen nach Anspruch 6, **dadurch gekennzeichnet, dass** sie als Komponente (b) UV-A-Filter enthalten, die ausgewählt sind aus der Gruppe, die gebildet wird von Benzoylmethan- und Enaminverbindungen sowie deren Gemischen.

8. Zubereitungen nach den Ansprüchen 6 und/oder 7, **dadurch gekennzeichnet, dass** sie als Komponente (b) UV-B-Filter enthalten, die ausgewählt sind aus der Gruppe, die gebildet wird von 3-Benzylidencampher bzw. 3-Benzylidennorcampher und dessen Derivaten, 4-Aminobenzoesäurederivaten, Estern der Zimtsäure, Estern der Salicylsäure, Derivaten des Benzophenons, Estern der Benzalmalonsäure, Triazinderivaten, Propan-1,3-dionen, Ketotricyclo(5.2.1.0)decan-Derivaten, 2-Phenylbenzimidazol-5-sulfonsäure und deren Salzen; Sulfonsäurederivaten von Benzophenonen, Sulfonsäurederivaten des 3-Benzylidencamphers sowie deren Gemischen.

9. Zubereitungen nach mindestens einem der Ansprüche 6 bis 8, **dadurch gekennzeichnet, dass** sie als Komponente (b) Pigmente enthalten, die ausgewählt sind aus der Gruppe, die gebildet wird von Zinkoxid, Titandioxid, Eisenoxid(en), Zirkoniumoxid, Siliziumdioxid, Manganoxid, Aluminiumoxid, Ceroxid, Silikaten, Bariumsulfat und Zinkstearat sowie deren Gemischen.

10. Zubereitungen nach mindestens einem der Ansprüche 6 bis 9, **dadurch gekennzeichnet, dass** sie als Komponente (c) Träger enthalten, die ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylenglycol(2)methylether, Propylpropylenglycol(3)methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkane, C14-C17 Alkane, C11-C15 Cycloalkane, Caprylylbutyrat, Isoparaffine, Diacetin, Triacetin Dicaprylylether, Dicaprylylmaleat, und deren Mischungen.

**11.** Zubereitungen mindestens einem der Ansprüche 6 bis 10, **dadurch gekennzeichnet, dass** sie weiterhin Ölkörper und/oder Emulgatoren enthalten.

**12.** Zubereitungen nach mindestens einem der Ansprüche 6 bis 11, **dadurch gekennzeichnet, dass** sie als Cremes, Lotionen, Gele, Pasten oder Kapseln vorliegen.

**13.** Zubereitungen nach mindestens einem der Ansprüche 6 bis 12, **dadurch gekennzeichnet, dass** es sich um ein Hautpflegemittel, ein Sonnenschutzmittel oder ein Haarpflegemittel handelt.

**14.** Nicht-therapeutisches Verfahren zur Inhibierung der durch Infrarotstrahlung induzierten Hautalterung, bei dem eine wirksame Menge einer Verbindung der Formel (I) oder deren Salze mit der menschlichen Haut oder den Haaren in Kontakt gebracht wird.

**15.** Verwendung von Verbindungen der Formel (I) oder deren Salze zur Inhibierung der durch Infrarotstrahlung induzierten Hautalterung.

Europäisches Patentamt
European Patent Office
Office européen des brevets

# EUROPÄISCHER RECHERCHENBERICHT

Nummer der Anmeldung
EP 13 15 6181

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | EP 1 591 105 A1 (STADA ARZNEIMITTEL AG [DE]) 2. November 2005 (2005-11-02)<br>* Absätze [0003] - [0010], [0020]; Ansprüche; Beispiele * | 1,2,14, 15<br>1-15 | INV.<br>A61K8/44<br>A61Q17/04 |
| X<br>Y | EP 2 545 898 A1 (COTY GERMANY GMBH [DE]) 16. Januar 2013 (2013-01-16)<br>* Absätze [0001], [0005], [0021] - [0026], [0039]; Ansprüche * | 1,2,4,6<br>1-15 | |
| X,D<br>Y | EP 2 181 697 A2 (SHISEIDO CO LTD [JP]) 5. Mai 2010 (2010-05-05)<br>* Absätze [0006], [0028]; Ansprüche; Tabellen 3-3 * | 1,3-13<br>1-15 | |
| X,D | WO 2010/124817 A2 (KPSS KAO GMBH [DE]; HOFFMANN MARTIN [DE]; GRIT MUSTAFA [DE]) 4. November 2010 (2010-11-04)<br>* Beispiele * | 1,3-6, 8-13 | |
| Y | NEELAM MUIZZUDDIN ET AL: "Effect of antioxidants and free radical scavengers on protection of human skin against UVB, UVA and IR irradiation", SKIN RESEARCH AND TECHNOLOGY, MUNKSGAARD, COPENHAGEN, DK, Bd. 5, 28. Dezember 1998 (1998-12-28), Seiten 260-265, XP002593991, ISSN: 0909-752X<br>* "abstract"; Seite 264, rechte Spalte, Absatz 4 * | 1-15 | RECHERCHIERTE SACHGEBIETE (IPC)<br>A61K<br>A61Q |
| X<br>Y | EP 1 310 238 A2 (BASF AG [DE]) 14. Mai 2003 (2003-05-14)<br>* Absätze [0001], [0009], [0116]; Ansprüche * | 1,3,4,6, 13<br>1-15 | |
| | -/-- | | |

Der vorliegende Recherchenbericht wurde für alle Patentansprüche erstellt

1

| Recherchenort | Abschlußdatum der Recherche | Prüfer |
|---|---|---|
| München | 22. Juli 2013 | Pregetter, Magdalena |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 13 15 6181

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| T | "Formulierungen", IP.COM JOURNAL, IP.COM INC., WEST HENRIETTA, NY, US, 26. Februar 2013 (2013-02-26), XP013156138, ISSN: 1533-0001 * das ganze Dokument * ----- | | |

RECHERCHIERTE SACHGEBIETE (IPC)

Der vorliegende Recherchenbericht wurde für alle Patentansprüche erstellt

1

| Recherchenort | Abschlußdatum der Recherche | Prüfer |
|---|---|---|
| München | 22. Juli 2013 | Pregetter, Magdalena |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 13 15 6181

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-07-2013

| Im Recherchenbericht angeführtes Patentdokument | Datum der Veröffentlichung | Mitglied(er) der Patentfamilie | Datum der Veröffentlichung |
|---|---|---|---|
| EP 1591105 A1 | 02-11-2005 | EP 1591105 A1 | 02-11-2005 |
| | | EP 2233127 A1 | 29-09-2010 |
| EP 2545898 A1 | 16-01-2013 | EP 2545898 A1 | 16-01-2013 |
| | | WO 2013007829 A2 | 17-01-2013 |
| EP 2181697 A2 | 05-05-2010 | AU 2007242342 A1 | 01-11-2007 |
| | | CN 101754744 A | 23-06-2010 |
| | | EP 2181697 A2 | 05-05-2010 |
| | | HK 1141709 A1 | 09-11-2012 |
| | | TW 200904477 A | 01-02-2009 |
| | | US 2010209365 A1 | 19-08-2010 |
| | | WO 2007122822 A2 | 01-11-2007 |
| WO 2010124817 A2 | 04-11-2010 | EP 2246033 A1 | 03-11-2010 |
| | | EP 2424489 A2 | 07-03-2012 |
| | | US 2012034182 A1 | 09-02-2012 |
| | | WO 2010124817 A2 | 04-11-2010 |
| EP 1310238 A2 | 14-05-2003 | CN 1418614 A | 21-05-2003 |
| | | DE 10155542 A1 | 22-05-2003 |
| | | EP 1310238 A2 | 14-05-2003 |
| | | JP 2003183145 A | 03-07-2003 |
| | | US 2003215405 A1 | 20-11-2003 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010124817 A2, Kao
- DE 10300782 A1, Beiersdorf
- WO 2007122822 A1, Shiseido
- WO 2003043431 A
- EP 1332772 A1


### In der Beschreibung aufgeführte Nicht-Patentliteratur


- **R.LOCHHEAD.** *Cosm.Toil.,* 1993, vol. 108, 95
- **SCHROEDER P ; LADEMANN J ; DARVIN ME ; STEGE H ; MARKS C ; BRUHNKE S ; KRUTMANN J.** Infrared radiation-induced matrix metalloproteinase in human skin. Implications for protection. *J Invest Dermatol,* 2008, vol. 128, 2491-2497